# EUROPÄISCHE PATENTSCHRIFT

(11) **EP 1 168 969 B1**
(45) Veröffentlichungstag und Bekanntmachung des Hinweises auf die Patenterteilung: **14.07.2004**
(21) Anmeldenummer: 00926809.5
(22) Anmeldetag: 04.04.2000
(51) Int. Cl.: A61B 17/32, B26F 3/00

(54) **WASSERSTRAHLEINRICHTUNG ZUM TRENNEN EINER BIOLOGISCHEN STRUKTUR**
WATER-JET DEVICE FOR SEPARATING A BIOLOGICAL STRUCTURE
DISPOSITIF A JET D'EAU POUR LA SEPARATION D'UNE STRUCTURE BIOLOGIQUE

(30) Priorität: 06.04.1999 DE 19915426
(43) Veröffentlichungstag der Anmeldung: 09.01.2002
(73) Patentinhaber: Andreas Pein Medizintechnik GmbH, 19061 Schwerin (DE)
(72) Erfinder: PEIN, Andreas, D-19061 Schwerin (DE)
(74) Vertreter: Jaap, Reinhard
(86) Internationale Anmeldenummer: PCT/EP2000/002994
(87) Internationale Veröffentlichungsnummer: WO 2000/059386

(56) Entgegenhaltungen:
- EP-A- 0 551 920
- EP-A- 0 691 183
- DE-C- 206 610
- US-A- 5 037 431

## Beschreibung

Die Erfindung bezieht sich auf eine Wasserstrahleinrichtung nach dem Oberbegriff des Anspruchs 1. Eine solche Einrichtung ist in der DE 4200976 beschrieben.

Wasserstrahleinrichtungen und ihre Anwendung in der Medizin sind in vielen Variationen bekannt. Ihr Vorteil gegenüber den mechanischen chirurgischen Geräten und Verfahren liegt in erster Linie in einem schonenderen Umgang mit den biologischen Strukturen.

Im allgemeinen haben solche Emrichtungen den Nachteil, dass die Trennflüssigkeit nicht steril gehalten werden kann und der Druck des austretenden Trennmediums relativ großen Schwankungen unterliegt.

In der DE 42 00 976 ist nun eine Wasserstrahlemrichtung beschrieben, die diese Nachteile abstellt und die im wesentlichen aus einer druckbelasteten Kolben-Zylinder-Einrichtung besteht, in dessen Zylinderraum ein Aufnahmebehälter für das Trennmedium eingepasst ist. Der Aufnahmebehälter für das Trennmedium ist dabei eine Kartusche und über eine Leitung mit einer Trenndüse verbunden. Durch die Trennung von Druckmedium und Trennmedium und durch die stabile Kartusche wird das Trennmedium steril gehalten und die Druckverhältnisse des Trennmediums bleiben konstant und reproduzierbar.

Das gewährleistet einen Wasserstrahl im laminaren Bereich und damit eine vorteilhafte Scharfkantigkeit des Wasserstrahls.

Aus der US 5 037 431 ist eine ähnliche Wasserstrahleinrichtung bekannt, die ebenfalls mit einer Trenndüse ausgerüstet ist, wobei die Trenndüse am distalen Ende der Versorgungsleitung angeordnet ist und einen zylindrischen Querschnitt besitzt.

Alle bisher bekannten Wasserstrahleinrichtungen haben aber den Nachteil, dass der Wasserstrahl gebündelt und mit seinem gesamten Querschnitt auf die biologische Struktur trifft. Zum Beispiel weiches Gewebe gibt diesem Druck leicht nach, so dass sich ein relativ breiter und auch oftmals unsauberer Trennschnitt ergibt. Dadurch wird die biologische Struktur über die Maßen strapaziert. Eine weitere Verfeinerung des Wasserstrahles durch eine weitere Verringerung des Düsendurchmessers ist technisch begrenzt. Ein solcher Wasserstrahl ist auch unter Flüssigkeit schwer einsetzbar, da er bereits kurz nach Austritt aus der Düse Auflösungserscheinungen zeigt und seine Trennschärfe verliert. Da obendrein die Trennkraft des Wasserstrahles ausschließlich durch die Größe des vom Operateur frei wählbaren Strömungsdruckes bestimmt wird. ist mit einer notwendigen Erhöhung des Strömungsdruckes in besonderen Fällen eine erhöhte Zerstörung der bilogischen Struktur verbunden.

Der Erfindung liegt die Aufgabe zu Grunde, die Trennschärfe bei Wasserstrahleinrichtungen der vorliegenden Art zu verbessern und weitestgehend unabhängig vom Strömungsdruck des Trennmediums zu halten.

Diese Aufgabe wird durch die kennzeichnenden Merkmale des Anspruchs 1 gelöst. Zweckdienliche Ausgestaltungen der Erfindung ergeben sich aus den Merkmalen der Ansprüche 2 bis 5.

Die Erfindung beseitigt die genannten Nachteile des Standes der Technik.

Von besonderem Vorteil ist die hohe Trenngenauigkeit des Wasserstrahles. Gegenüber einem laminaren und gerade gerichteten Wasserstrahl wird die Trenngenauigkeit durch den neuartigen und gedrehten Wasserstrahl weiter erhöht. Das wird dadurch erreicht. dass die Wasserteilchen durch die Drehung des Wasserstrahles in den äußeren Umfangsbereich des Wasserstrahles verlagert werden, wo sie gegenüber den mittig verbleibenden Wasserteilchen eine erhöhte Umfangsgeschwindigkeit einnehmen und so eine im Umfangsbereich umlaufende Schneidkante ausbilden. Diese umlaufende Schneidkante ist mit der umlaufenden Schneide eines Holzbohrers oder einer Lochkreissäge vergleichbar und trennt wegen der geringeren Angriffsfläche der Trennkante genauer als die volle Angriffsfläche eines geraden Wasserstrahles. Mit der verbesserten Trennwirkung kann der Flüssigkeitsdruck verringert werden, was sich für die gesamte Wasserstrahleinrichtung energetisch vorteilhaft auswirkt. Der so ausgebildete Wasserstrahl bohrt sich nicht nur sehr gut in die verschiedensten biologischen Strukturen, sondern auch in Flüssigkeiten ein. Damit ist der neue Wasserstrahl wegen seiner Kohärenzerhaltung auch hervorragend für Operationen unter Flüssigkeiten geeignet.

Von Vorteil ist auch, dass die geringere axiale Kraftkomponente des Strömungsdruckes auch eine geringere, als Rückstoß spürbare Gegenkraft erzeugt. Das erleichtert die Operation, da der Operateur auf den Abstand der Trenndüse vom Gewebe nicht mehr zu achten hat. Dagegen bleibt dem Operateur ohne nachteilige Auswirkungen das Gespür für die momentane Trennkraft erhalten, da ihm die momentane Strömungskraft durch die auf den Handgriff wirkende Gegenkraft der radialen Kraftkomponente spürbar gemacht wird. Das alles erhöht die Konzentrationsfähigkeit des Operateurs.

Der neue gedrehte Wasserstrahl bildet sich durch die Verhältnismäßigkeit von Durchmesser und Länge des Düsenkanals äußerst scharfkantig aus. Es ist für die Scharlkantigkeil des Wasserstrahles auch sehr vorteilhaft, wenn dazu eine Steigung der Drallnuten gewählt wird, die größer als der Durchmesser des Düsenkanals ist.

Für die Ausbildung eines scharfkantigen Wasserstrahles ist es auch von Vorteil, wenn eine gerundete Querschnittsform der Drallnuten gewählt wird.

Es hat sich als zweckmäßig erwiesen, dass die Versorgungskapillare als stumpfes Trennwerkzeug unterstützend zum Wasserstrahl eingesetzt wird, das erspart weitestgehend eine scharfe Präparation des Gewebes und vermeidet damit stärkere Blutungen. Die neue Versorgungskapillare ist auch mit anderen mechanischen Trennwerkzeugen kombinierbar. Das erweitert den Einsatzbereich der Wasserstrahleinrichtung.

Von besonderem Vorteil ist, wenn die Versorgungskapillare aus einem stromleitfähigen Material besteht und mit einer hochfrequenten monopolaren oder bipolaren Stromversorgungseinrichtung vcrbindbar ist. Damit können zum Beispiel in einem Expansionsraum zwischen unterschiedlichen Gewebestrukturen solche dünnen Gewebefäden durch Hitze zertrennt werden, die einem Wasserstrahl ausweichen würden.

Die Erfindung soll nachstehend an Hand eines Ausführungsbeispieles näher erläutert werden.

Dazu zeigen
- Fig. 1:: eine vereinfacht und sinnbildlich dargestellte Wasserstrahleinrichtung,
- Fig. 2.: eine Präzisionsdüse im Teilschnitt und
- Fig. 3:: die Präzisionsdüse in der Vorderansicht.

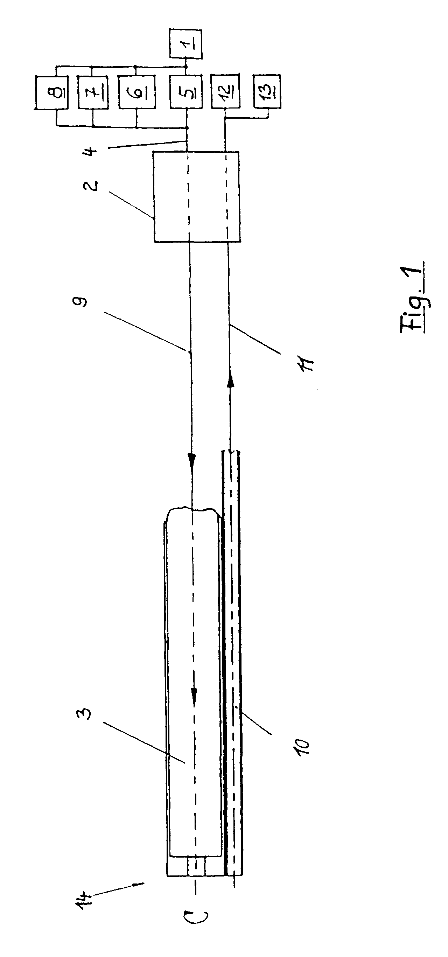

Nach der Fig. 1 besteht die Wasserstrahleinrichtung in der Hauptsache aus einem Druckstromerzeuger 1, einer Steuer- und Regeleinheit 2 und einer Versorgungskapillare 3 mit einem Handgriff. In der vom Druckstromerzeuger 1 zur Steuer- und Regeleinheit 2 führenden Druckleitung 4 ist ein zuschaltbarer Pulserzeuger 5 zwischengeschaltet, zu dem weitere Zusatzeinrichtungen wie beispielsweise ein Lasergerät 6, eine Heizeinrichtung 7 und/oder ein Vereisungsgerät 8 parallel geschaltet sind. Die Steuer- und Regeleinheit 2 ist mit der Versorgungskapillare 3 ausgerüstet und besitzt nicht dargestellte Betätigungseinrichtungen, mit Hilfe derer der Operateur alle den Wasserstrahl beeinflussenden Parameter vorwählen oder stufenlos regeln kann. Die Steuer- und Regeleinheit 2 ist über eine Versorgungsleitung 9 mit der Versorgungskapillare 3 verbunden. Parallel zur Versorgungskapillare 3 kann eine Entsorgungskapillare 10 vorgesehen sein, die in baulicher Einheit oder in separater Ausführung zur Versorgungskapillare 3 ausgeführt sein kann und die über eine Entsorgungsleitung 11 mit der Steuer- und Regeleinheit 2 Verbindung hat. Eine regelbare Entsorgungspumpe 12 sorgt für den erforderlichen Unterdruck. Parallel zur Entsorgungspumpe 12 befinden sich ein oder mehrere steuer- und regelbare Zusatzeinheiten 13.

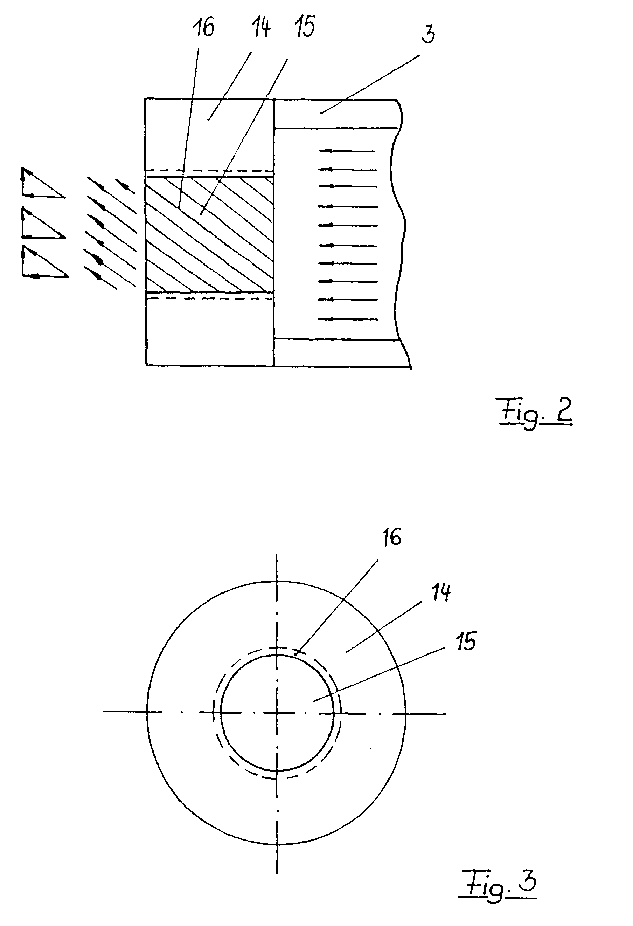

Wie die Fig. 2 und 3 zeigen, ist die Versorgungskapillare 3 an ihrem distalen Ende mit einer erfindungsgemäßen Trenndüse 14 ausgerüstet. Diese Trenndüse 14 besitzt einen Düsenkanal 15, der in besonderer Weise mit einer oder mehreren parallelen und umlaufenden Drallnuten 16 ausgerüstet ist. Die Drallnuten 16 können eine beliebige Querschnittsform aufweisen, wobei eine runde Querschnittsform vorteilhaft ist. Aus strömungstechnischer Sicht ist das Verhältnis der Steigung der Drallnuten 16 zum Durchmesser des Düsenkanals 15 größer als eins gewählt.

Die Versorgungskapillare 3 ist in Verbindung mit der Trenndüse 14 so ausgelegt, dass das distale Ende der Versorgungskapillare 3 als ein zusätzlich mechanisch wirkendes Trennmittel einsetzbar ist.

Die Wirkungsweise der Wasserstrahleinrichtung soll an Hand der Hydrodissektionsmethode beschrieben werden. Dabei wird zunächst die Wasserstrahleinrichtung in Betriebsbereitschaft gebracht, so dass ein Wasserstrahl mit entsprechend vorprogrammierten Druck, Menge und Temperatur abrufbereit zur Verfügung steht. Danach wird die Versorgungskapillare 3 in das Gewebe eingestochen und in den Grenzschichtbereich unterschiedlicher Gewebe geführt. Anschließend wird über die Versorgungskapillare 3 Flüssigkeit in diesen Bereich appliziert, wodurch sich zwischen den unterschiedlichen Geweben ein Expansionsraum bildet, der das Gewebe auseinander drückt. Dabei werden weiche Gewebebestandteile bereits bei geringsten Drücken disseziert, harte oder elastische Strukturen straffen sich und bleiben zunächst noch unverletzt. Bei sehr fest aneinanderliegenden Strukturen kann dieser Prozess durch eine Pulsierung des Wasserstrahles unterstützt werden.

Der Wasserstrahl hat dabei eine besondere Wirkung. Durch die im Düsenkanal 15 der Trenndüse 14 befindlichen Drallnuten 16 wird die laminare Strömung des Wasserstrahls umgelenkt und eine Drehbewegung in Umfangsrichtung eingeleitet. Dadurch wird die in die Trenndüse 14 eingeleitete Strömungskraft des Wasserstrahls in eine axial verbleibende und in eine radial dazukommende Kraftkomponente aufgeteilt. Es bildet sich ein gedrehter Wasserstrahl, bei dem die laminare Strömung erbleibt, da die Bewegungsbahnen der einzelnen Wasserteilchen weiterhin parallel zueinander verlaufen. Die radial wirkende Kraftkomponente wirkt auf den Wasserstrahl ein und verlagert die Wasserteilchen verstärkt in den umfangsnahen Bereich, wo sie sich mit einer erhöhten Umfangsgeschwindigkeit bewegen. Dadurch bildet sich in diesem Bereich des Wasserstrahles eine geschlossene umlaufende Trennkante in einer zu einem Holzbohrer vergleichbaren Ausbildung. Diese Trennkante hat naturgemäß gegenüber einem geraden Wasserstrahl eine erhöhte Trennkraft.

Im Bedarfsfalle wird die über die Versorgungskapillare 3 eingelassene Wassermenge über die Entsorgungskapillare 10 aus dem Gewebebereich wieder abgezogen.

### Aufstellung der Bezugszeichen

- 1: Druckstromerzeuger
- 2: Steuer und Regeleinheit
- 3: Versorgungskapillare
- 4: Druckleitung
- 5: Pulserzeuger
- 6: Lasergerät
- 7: Heizeinrichtung
- 8: Vereisungsgerät
- 9: Versorgungsleitung
- 10: Entsorgungskapillare
- 11: Entsorgungsleitung
- 12: Entsorgungspumpe
- 13: Zusatzeinheit
- 14: Trenndüse
- 15: Düsenkanal
- 16: Drallnut

## Patentansprüche

1. Chirurgische Wasserstrahleinrichtung zum Trennen einer biologischen Struktur, im wesentlichen bestehend aus einem Druckstromerzeuger (1), einer bedienbaren Steuer- und Regeleinheit (2) und einer Versorgungskapillare (3) mit einer Trenndüse (14), aus der der Trennstrahl austritt, wobei die Trenndüse (14) einen Düsenkanal (15) mit einem zylindrischen Abschnitt besitzt und sich am distalen Ende der Versurgungskapillare (3) befindet, wobei die Trenndüse (14), feststehend und koaxial zur Versorgungskapillare (3) angeordnet ist, **dadurch gekennzeichnet, dass** der Düsenkanal (15) am Umfang mit mindestens einer Drallnut (16) ausgerüstet ist und die Anzahl der Drallnuten (16) und der Durchmesser und die Länge des Düsenkanals (15) in einem solchen Verhältnis zueinander stehen, dass der unter Druck stehende Trennstrahl gedreht wird.

2. Wasserstrahleinrichtung nach Anspruch 1,
**dadurch gekennzeichnet, dass** die Steigung der Drallnuten (16) größer als der Durchmesser des Düsenkanals (15) ausgelegt ist.

3. Wasserstrahleinrichtung nach Anspruch 2,
**dadurch gekennzeichnet, dass** die Drallnuten (16) eine gerundete Querschnittsform aufweisen.

4. Wasserstrahleinrichtung nach Anspruch 1,
**dadurch gekennzeichnet, dass** die Versorgungskapillare (3) im Bereich ihrer Trenndüse (14) so ausgelegt ist, dass das distale Ende der Versorgungskapillare als ein zusätzlich mechanisch wirkendes Trennmiltel einsetzbar ist.

5. Wasserstrahleinrichtung nach Anspruch 1,
**dadurch gekennzeichnet, dass** die Versorgungskapillare (3) aus einem stromleitfähigen Material besteht und mit einer hochfrequenten Stromversorgungseinrichtung verbindbar ist.

## Claims

1. Surgical water-jet device for separating a biological structure, substantially consisting of a pressure current generator (1), an operable control and regulating unit (2) and a supply capillary (3) with a separating nozzle (14), from which the separating jet emerges, wherein the separating nozzle (14) has a nozzle channel (15) with a cylindrical portion and is located at the distal end of the supply capillary (3), wherein the separating nozzle (14) is stationary and disposed coaxially with the supply capillary (3), **characterised in that** the nozzle channel (15) is provided at the circumference with at least one helical groove (16), and the number of helical grooves (16) and the diameter and the length of the nozzle channel (15) are related to one another such that the pressurised separating jet is rotated.

2. Water-jet device according to Claim 1,
**characterised in that** the pitch of the helical grooves (16) is greater than the diameter of the nozzle channel (15).

3. Water-jet device according to Claim 2,
**characterised in that** the helical grooves (16) have a rounded cross-sectional shape.

4. Water-jet device according to Claim 1,
**characterised in that** the supply capillary (3) is designed in the region of its separating nozzle (14) such that the distal end of the supply capillary can be used as a separating means acting in an additional mechanical manner.

5. Water-jet device according to Claim 1,
**characterised in that** the supply capillary (3) consists of a conductive material and can be connected to a highfrequency power supply device.

## Revendications

1. Dispositif à jet d'eau chirurgical pour la séparation d'une structure biologique, comprenant pour l'essentiel un générateur de courant de pression (1), une unité de commande et de régulation (2) pouvant être actionnée et un capillaire d'alimentation (3) avec une buse de séparation (14) par laquelle sort le jet de séparation, la buse de séparation (14) présentant un canal de buse (15) avec une portion cylindrique, et étant montée fixe et coaxiale sur l'extrémité distale du capillaire d'alimentation (3),
**caractérisé en ce qu'**
à la périphérie le canal de buse (15) est muni d'au moins une rainure de rotation (16), et le nombre des rainures de rotation (16) ainsi que le diamètre et la longueur du canal de buse (15) présentent un tel rapport réciproque que le jet de séparation sous pression est mis en rotation.

2. Dispositif à jet d'eau selon la revendication 1,
**caractérisé en ce que**
le pas des rainures de rotation (16) est supérieur au diamètre du canal de buse (15).

3. Dispositif à jet d'eau selon la revendication 2,
**caractérisé en ce que**
les rainures de rotation (16) présentent une forme de section transversale arrondie.

4. Dispositif à jet d'eau selon la revendication 1,
**caractérisé en ce que**
dans la zone de sa buse de séparation (14) le capillaire d'alimentation (3) est conçu de telle sorte que l'extrémité distale du capillaire d'alimentation peut être utilisée comme moyen de séparation à action mécanique supplémentaire.

5. Dispositif à jet d'eau selon la revendication 1,
**caractérisé en ce que**
le capillaire d'alimentation (3) est réalisé en un matériau conducteur de courant et peut être relié à une installation d'alimentation en courant haute fréquence.
